(19) Europäisches Patentamt
European Patent Office
Office européen des brevets

(11) Veröffentlichungsnummer: **0 142 467 B1**

# (12) EUROPÄISCHE PATENTSCHRIFT

(45) Veröffentlichungstag der Patentschrift: **24.08.88**

(51) Int. Cl.⁴: **C 07 D 401/14**, C 07 D 471/10, C 08 K 5/34

(21) Anmeldenummer: **84810455.0**

(22) Anmeldetag: **17.09.84**

## (54) Piperidylreste enthaltende polymere Verbindungen und ihre Verwendung als Stabilisatoren für synthetische Polymere.

(30) Priorität: **23.09.83 IT 2297683**

(43) Veröffentlichungstag der Anmeldung: **22.05.85 Patentblatt 85/21**

(45) Bekanntmachung des Hinweises auf die Patenterteilung: **24.08.88 Patentblatt 88/34**

(84) Benannte Vertragsstaaten: **DE FR GB IT**

(56) Entgegenhaltungen:
**EP - A - 0 003 542**
**FR - A - 2 521 143**
**US - A - 4 028 334**
**US - A - 4 250 268**

(73) Patentinhaber: **CIBA-GEIGY S.p.A., Strada Statale 233-Km. 20,5, Origgio (IT)**
(84) Benannte Vertragsstaaten: **IT**

(73) Patentinhaber: **CIBA-GEIGY AG, Klybeckstrasse 141, CH-4002 Basel (CH)**
(84) Benannte Vertragsstaaten: **DE FR GB**

(72) Erfinder: **Cantatore, Giuseppe, Dr., Via Generale Planelli, 68, I-70032 Bitonto (Bari) (IT)**

(74) Vertreter: **Stamm, Otto et al, Patentabteilung der CIBA-GEIGY AG Postfach, CH-4002 Basel (CH)**

EP 0 142 467 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Piperidylreste enthaltende polymere Verbindungen, welche als Stabilisatoren für synthetische Polymere gegen lichtinduzierten, thermischen oder oxidativen Abbau verwendet werden können.

Hydroxygruppen enthaltende Polyether mit Polyalkylpiperidylaminogruppen sind in US Patent Nr. 4 294 963 als Stabilisatoren für Polymere beschrieben. Neue polymere Triazin-piperidylester sind nun gefunden worden, die in synthetischen Polymeren eine ausserordentliche Lichtschutzwirkung zeigen.

Die FR-A-2 521 143 beschreibt monomere Triazinderivate, die Piperidylreste enthalten, sowie eine Verwendung dieser Verbindungen zum Stabilisieren von Polymeren.

Demnach betrifft die vorliegende Erfindung Verbindungen der Formel I

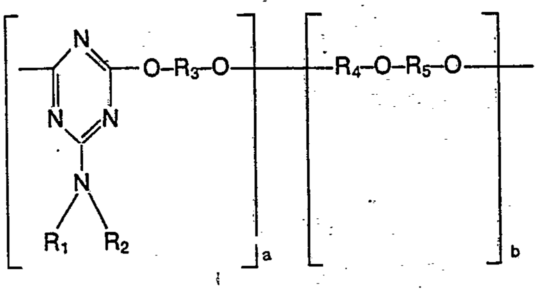
(I)

worin $R_1$ und $R_2$ unabhängig voneinander Wasserstoff, $C_1$–$C_{18}$ Alkyl, 2-Hydroxyethyl, 3-Hydroxypropyl, 2-Methoxyethyl, 2-Ethoxyethyl, 3-Methoxypropyl, 3-Ethoxypropyl, 3-Octyloxypropyl, 3-Dodecyloxypropyl, 3-Octadecyloxypropyl, 3-Dimethylaminopropyl, 3-Diethylaminopropyl, 4-Diethylaminobutyl, $C_3$–$C_{18}$ Alkenyl, $C_5$–$C_{18}$ Cycloalkyl, $C_6$–$C_{18}$ Aryl, $C_7$–$C_{18}$ Aralkyl oder eine Gruppe der Formel II

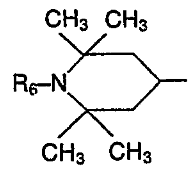
(II)

bedeuten, worin $R_6$ Wasserstoff, $C_1$–$C_{12}$ Alkyl, $C_3$–$C_{12}$ Alkenyl oder Alkynyl, $C_7$–$C_{12}$ Aralkyl oder $C_1$–$C_{12}$ Acyl ist, oder $R_1$ und $R_2$ zusammen mit dem Stickstoffatom, an dem sie gebunden sind, einen heterocyclischen Rest ausgewählt aus der Gruppe bestehend aus Pyrrolidin-1-yl, Piperidin-1-yl, Hexahydroazepin-1-yl, Morpholin-4-yl und 4-Methylpiperazin-1-yl bilden, $R_3$ und $R_5$ unabhängig voneinander eine der Gruppen der Formeln III, IV, V oder VI

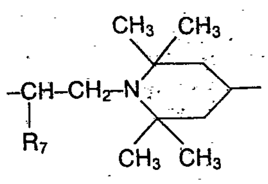
(III)

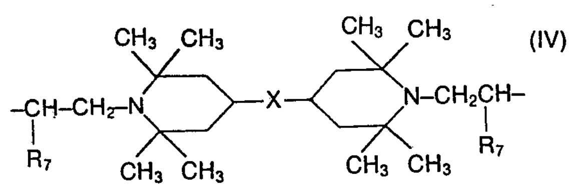
(IV)

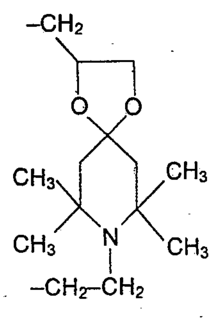
(V)

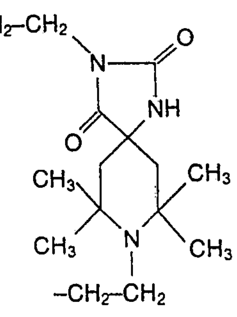
(VI)

bedeuten und $R_5$ zusätzlich eine Gruppe der Formel VII

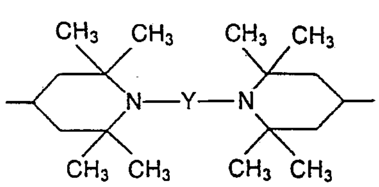
(VII)

sein kann, worin $R_7$ Wasserstoff, $C_1$–$C_6$ Alkyl oder Phenyl ist, X eine Gruppe $-O-(CH_2)_n-O-$ oder $-N(R_8)-C(O)-(CH_2)_n-C(O)-N(R_8)-$ bedeutet, n eine Zahl von 1 bis 6 ist, $R_8$ die gleiche Bedeutung wie $R_1$ und $R_2$ hat und Y $C_2$–$C_{12}$ Alkylen, durch eine Hydroxygruppe substituiertes oder durch ein Sauerstoffatom unterbrochenes $C_3$–$C_{12}$ Alkylen, $C_4$–$C_{12}$ Alkenylen oder $C_3$–$C_{12}$ Aralkylen bedeutet, $R_4$ $C_2$–$C_{18}$ Diacyl oder eine Gruppe der Formel VIII

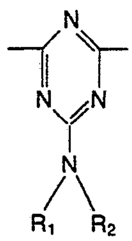

(VIII)

ist, worin $R_1$ und $R_2$ die oben angegebene Bedeutung haben, a eine Zahl zwischen 0,2 und 1 und b eine Zahl zwischen Null und 0,8 sind, wobei die Verbindungen der Formel I ein mittleres Molekulargewicht $\overline{M}n$ zwischen 1000 und 20 000 aufweisen.

Die Bedeutungen der verschiedenen Reste speziell erläuternde Beispiele sind wie folgt:

Für $R_1$, $R_2$ und $R_8$:
Wasserstoff, Methyl, Ethyl, Propyl, Isopropyl, Butyl, But-2-yl, Isobutyl, Hexyl, 2-Ethylhexyl, Octyl, Decyl, Dodecyl, Hexadecyl, Octadecyl, 2-Hydroxyethyl, 3-Hydroxypropyl, 2-Methoxyethyl, 2-Ethoxyethyl, 3-Methoxypropyl, 3-Ethoxypropyl, 3-Octyloxypropyl, 3-Dodecyloxypropyl, 3-Octadecyloxypropyl, 3-Dimethylaminopropyl, 3-Diethylaminopropyl, 4-Diethylaminobutyl, Allyl, Methallyl, But-2-enyl, Undec-10-enyl, Oleyl, Cyclohexyl, Methylcyclohexyl, Trimethylcyclohexyl, Cyclooctyl, Cyclododecyl, Phenyl, Methylphenyl, Dimethylphenyl, Trimethylphenyl, t-Butylphenyl, t-Octylphenyl, Methoxyphenyl, Ethoxyphenyl, 3,5-Di-t-butyl-4-hydroxyphenyl, Benzyl, Methylbenzyl, Hydroxybenzyl, 3,5-Di-t-butyl-4-hydroxybenzyl, 2,2,6,6-Tetramethyl-piperidin-4-yl, 1,2,2,6,6-Pentamethyl-piperidin-4-yl, 1-Allyl-2,2,6,6-tetramethyl-piperidin-4-yl, 1-Benzyl-2,2,6,6-tetramethyl-piperidin-4-yl und 1-Acetyl-2,2,6,6-tetramethyl-piperidin-4-yl;

Für $R_4$:
Oxalyl, Malonyl, Succinyl, Glutaryl, Adipoyl, Sebacoyl, Isophthaloyl, Terephthaloyl oder eine Gruppe der Formel (V), worin $R_1$ und $R_2$ die oben angegebene Bedeutung haben;

Für $R_6$:
Wasserstoff, Methyl, Ethyl, Propyl, Butyl, Hexyl, Octyl, Decyl, Dodecyl, Allyl, Methallyl, But-2-enyl, Hex-2-enyl, Propargyl, Benzyl, Methylbenzyl, t-Butylbenzyl, Hydroxybenzyl, Acetyl, Propionyl, Butyryl, Caproyl und Benzoyl;

Für $R_7$:
Wasserstoff, Methyl, Ethyl, Propyl, Butyl, Hexyl und Phenyl,

Für Y:
Ethylen, Trimethylen, Tetramethylen, Hexamethylen, Octamethylen, Decamethylen, Dodecamethylen, 2-Hydroxypropan-1,3-diyl, 3-Oxapentan-1,5-diyl, But-2-en-1,4-diyl und Xylylen.

Von besonderem Interesse sind die Verbindungen der Formel I, worin $R_1$ und $R_2$ unabhängig voneinander
Wasserstoff, $C_1$–$C_{12}$ Alkyl, 2-Methoxyethyl, 2-Ethoxyethyl, 3-Methoxypropyl, 3-Ethoxypropyl, 3-Octyloxypropyl, 3-Dodecyloxypropyl, $C_3$–$C_6$ Alkenyl, $C_6$–$C_{10}$ Cycloalkyl
oder eine Gruppe der Formel II bedeuten, worin $R_6$ Wasserstoff, Methyl, Allyl, Benzyl oder Acetyl ist, oder $R_1$ und $R_2$ zusammen mit dem Stickstoffatom an dem sie gebunden sind Pyrrolidin-1-yl, Piperidin-1-yl, Hexahydroazepin-1-yl oder Morpholin-4-yl bilden, $R_3$ und $R_5$ eine Gruppe der Formel III sind und $R_5$ zusätzlich eine Gruppe der Formel VII sein kann, $R_7$ Wasserstoff, Methyl oder Ethyl ist, Y $C_2$–$C_6$ Alkylen, But-2-en-1,4-diyl oder Xylylen bedeutet, $R_4$ $C_2$–$C_{12}$ Diacyl oder eine Gruppe der Formel VIII ist, worin $R_1$ und $R_2$ die oben angegebene Bedeutung haben, a eine Zahl zwischen 0,3 und 1 und b eine Zahl zwischen Null und 0,7 bedeuten, wobei die Verbindungen der Formel I ein mittleres Molekulargewicht $\overline{M}n$ zwischen 1500 und 10 000 aufweisen.

Bevorzugt sind Verbindungen der Formel I, worin $R_1$ und $R_2$ unabhängig voneinander
$C_1$–$C_8$ Alkyl, Cyclohexyl,
2,2,6,6-Tetramethylpiperidin-4-yl oder
1,2,2,6,6-Pentamethylpiperidin-4-yl
bedeuten, $R_3$ und $R_5$ eine Gruppe der Formel III sind und $R_5$ zusätzlich eine Gruppe der Formel VII sein kann, $R_7$ Wasserstoff ode Methyl ist, Y But-2-en-1,4-diyl oder Xylylen bedeutet, a die Zahl 1 und b Null bedeuten, wobei die Verbindungen der Formel I ein mittleres Molekulargewicht $\overline{M}n$ zwischen 1500 und 6000 aufweisen.

Verbindungen der Formel I, worin b nicht Null bedeutet, können hergestellt werden durch Umsetzung einer Mischung von Verbindungen der Formeln IX und X

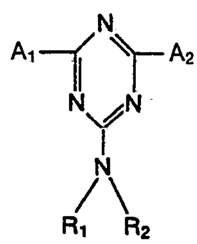

(IX)

$A_3$–$R_4$–$A_4$ ,

(X)

worin $R_1$, $R_2$ und $R_4$ die oben angegebene Bedeutung haben und $A_1$, $A_2$, $A_3$ und $A_4$ Cl oder $C_1$–$C_4$ Alkoxy bedeuten, mit einer der Verbindungen der folgenden Formeln XI, XII, XIII oder XIV

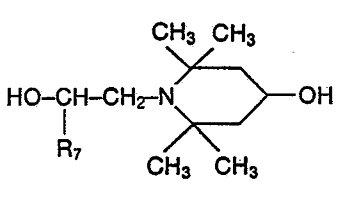

(XI)

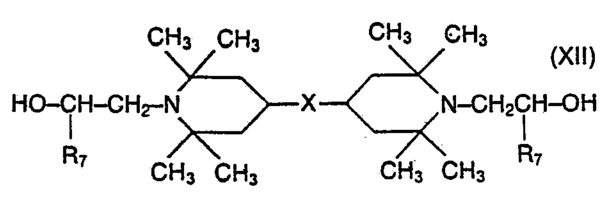

(XII)

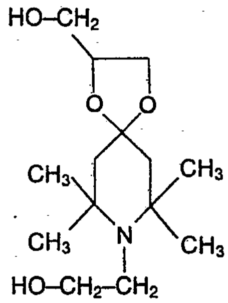

(XIII)

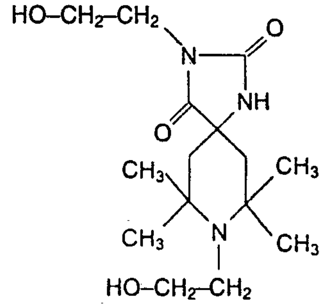

(XIV)

und, wenn $R_5$ eine Gruppe der Formel VII ist, zusätzlich mit einer Verbindung der Formel XV

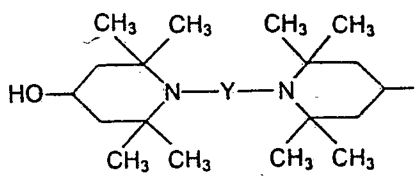

(XV),

worin $R_7$, X und Y die oben angegebene Bedeutung haben, vorzugsweise im stöchiometrischen Verhältnis.

Wenn b Null ist, werden die Verbindungen der Formel I durch Umsetzung einer Verbindung der Formel IX mit einer der Verbindungen der Formeln XI, XII, XIII oder XIV und gegebenenfalls XV in annähernd stöchiometrischem Verhältnis hergestellt.

In beiden Fällen kann die Reaktion ohne oder mit einem inerten organischen Lösungsmittel durchgeführt werden, in Gegenwart einer organischen oder anorganischen Base, wenn $A_1$, $A_2$, $A_3$ und $A_4$ Cl bedeuten, oder eines Umesterungskatalysators, wenn $A_1$, $A_2$, $A_3$ und $A_4$ Alkoxy sind.

Als Lösungsmittel eignen sich z.B. Benzol, Toluol, Xylol, Ethylbenzol, Trimethylbenzol, Tetralin, Decalin, Octan, Decan, Gemische von aliphatischen Kohlenwasserstoffen mit Siedepunkt zwischen 100 und 200 °C,
Dioxan, Dibutylether, 1,2-Dimethoxyethan,
1,2-Diethoxyethan, Diethylenglykol-dimethylether und Diethylenglykoldiethyleter,
N,N-Dimethylformamid, N,N-Dimethylacetamid,
N-Methylpyrrolidon und Dimethylsulfoxid.

Geeignete Basen, die (bei $A_1$, $A_2$, $A_3$ und $A_4$ = Cl) zum Abfangen der sich bei der Reaktion bildenden Halogenwasserstoffsäure eingesetzt werden, sind z.B.
Pyridin, Triethylamin, Tributylamin,
Natriumhydroxid, Kaliumhydroxid,
Natriumcarbonat, Kaliumcarbonat.
In jedem Fall wird die Base in mindestens der äquivalenten Menge der durch die Reaktion frei werdenden Halogenwasserstoffsäure eingesetzt.

Für die Fälle in denen $A_1$, $A_2$, $A_3$ und $A_4$ Alkoxy bedeuten, können als Umesterungskatalysatoren z.B. Alkalimetalle sowie deren Alkoholate, Hydride und Amide eingesetzt werden.

Die Polykondensation kann bei Temperaturen zwischen 80 und 280 °C, bevorzugt zwischen 100 und 200 °C im molaren Verhältnis Verbindung der Formel IX + Verbindung der Formel X: Verbindung der Formel XI, XII, XIII oder XIV + Verbindung der Formel XV zwischen 1,3:1 und 1:1,3, bevorzugt zwischen 1,2:1 und 1:1,2, erfolgen.

Die verschiedenen Edukte können gleichzeitig oder nacheinander umgesetzt werden.

Die Verbindungen der Formel IX können in Analogie zu bekannten Verfahren, ausgehend von Cyanurchlorid, erhalten werden. Die erhaltenen Dichlortriazine oder Dialkoxytriazine können direkt, ohne Isolierung von der Reaktionsmischung, oder nach Isolierung zur weiteren Reaktion eingesetzt werden. Die Verbindungen der Formeln XI, XII, XIII, XIV und XV sind bekannte Verbindungen.

Wie anfangs erwähnt, sind die Verbindungen der Formel I sehr wirksam bei der Verbesserung der Licht-, Wärme- und Oxydationsbeständigkeit von synthetischen Polymeren, wie beispielsweise
Polyethylen hoher oder niedriger Dichte,
Polypropylen, Ethylen/Propylencopolymeren,
Ethylen/Vinylacetatcopolymeren, Polybutadien,
Polyisopren, Polystyrol,
Butadien/Styrolcopolymeren,
Acrylnitril/Butadien/Styrolcopolymeren,
Polymeren und Copolymeren von
Vinylchlorid und Vinylidenchlorid,
Polyoxymethylen, Polyurethanen,
gesättigten und ungesättigten Polyestern,
Polyamiden, Polycarbonaten, Polyacrylaten,
Alkydharzen und Epoxidharzen.

Die Verbindungen der Formel I können im Gemisch mit synthetischen Polymeren in verschiedenen Anteilen eingesetzt werden, die von der Art des Polymeren, dem Endzweck und der Gegenwart weiterer Zusatzstoffe abhängen. Im allgemeinen setzt man zweckmässig 0,01 bis 5 Gew.-% der Verbindung der Formel I, bezogen auf das Gewicht der Polymeren, vorzugsweise 0,05 bis 1%, ein.

Die Verbindungen der Formel I können nach verschiedenen Verfahren in die polymeren Materialien eingearbeitet werden, wie durch trockenes Einmischen in Pulverform oder nasses Einmischen in Form einer Lösung oder Suspension oder auch in Form eines konzentrierten Vorgemisches; das synthetische Polymer lässt sich dabei in Form von Pulver, Granulat, Lösung, Suspension oder Latex einsetzen. Die mit den Produkten der Formel I stabilisierten Polymeren lassen sich zur Herstellung von Formkörpern, Folien, Bändern, Fasern, Endlosfäden, Lacken usw. verwenden.

Gegebenenfalls kann man den Mischungen von Verbindungen der Formel I mit den synthetischen Polymeren weitere Zusatzstoffe beigeben, wie

sein kann, $R_7$ Wasserstoff oder Methyl ist, Y But-2-en-1,4-diyl oder Xylylen bedeutet, a die Zahl 1 und b Null bedeuten, wobei die Verbindungen der Formel I ein mittleres Molekulargewicht $\overline{Mn}$ zwischen 1500 und 6000 aufweisen.

4. Polymerzusammensetzungen, dadurch gekennzeichnet, dass sie ein synthetisches Polymer und eine Stabilisatorverbindung der Formel I gemäss Anspruch 1 in einer Menge zwischen 0,01 und 5 Gew.-%, bezogen auf das Gewicht der synthetischen Polymers, enthalten.

5. Zusammensetzungen nach Anspruch 4, dadurch gekennzeichnet, dass sie zusätzlich zu dem neuen Stabilisator weitere übliche Zusatzstoffe für synthetische Polymere enthalten.

6. Zusammensetzungen nach Anspruch 4, dadurch gekennzeichnet, dass als synthetisches Polymer Polyäthylen oder Polypropylen vorliegt.

**Claims**

1. A compound of the formula I

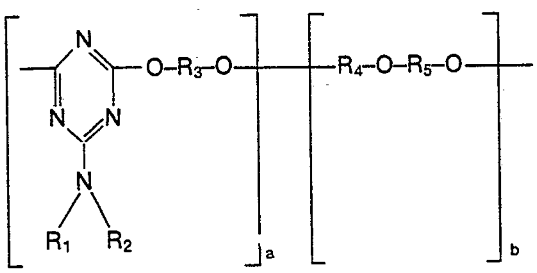

(I)

in which $R_1$ and $R_2$ independently of one another are
hydrogen, $C_1C_{18}$alkyl, 2-hydroxyethyl, 3-hydroxypropyl, 2-methoxyethyl, 2-ethoxyethyl, 3-methoxypropyl, 3-ethoxypropyl, 3-octyloxypropyl, 3-dodecyloxypropyl, 3-octadecyloxypropyl, 3-dimethylaminopropyl, 3-diethylaminopropyl, 4-di-ethylaminobutyl, $C_3$–$C_{18}$alkenyl, $C_5$–$C_{18}$cycloalkyl, $C_6$–$C_{18}$aryl, $C_7$–$C_{18}$aralkyl
or a group of the formula II

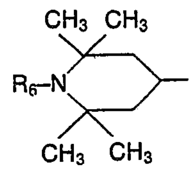

(II)

in which $R_6$ is hydrogen, $C_1$–$C_{12}$alkyl, $C_3$–$C_{12}$alkenyl or alkynyl, $C_7$–$C_{12}$aralkyl or $C_1$–$C_{12}$acyl, or $R_1$ and $R_2$, together with the nitrogen atom to which they are bonded, form a heterocyclic radical selected from the group comprising
pyrrolidin-1-yl, piperidin-1-yl, hexahydroazepin-1-yl, morpholin-4-yl or 4-methylpiperazin-1-yl,
$R_3$ and $R_5$ independently of one another denote one of the groups of the formulae III, IV, V or VI

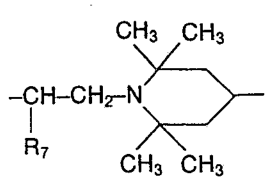

(III)

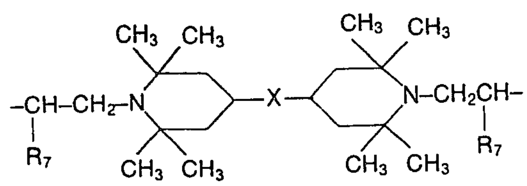

(IV)

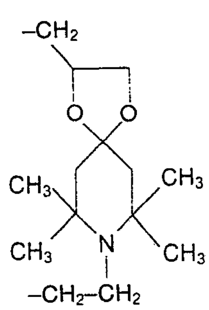

(V)

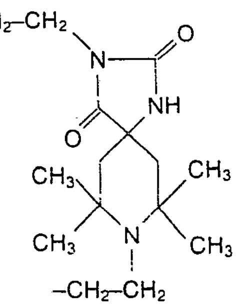

(VI)

and $R_5$ additionally can be a group of the formula VII

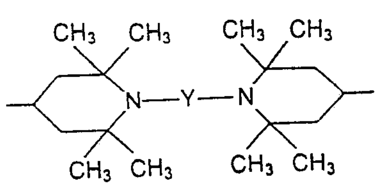

(VII)

in which $R_7$ is hydrogen, $C_1$–$C_6$alkyl or phenyl, X is a group
$O-(CH_2)_n-O-$ or $-N(R_8)-C(O)-(CH_2)_nC(O)-N(R_8)-$,
n is a number from 1 to 6, $R_8$ has the same meaning as $R_1$ and $R_2$ and Y is $C_2$–$C_{12}$alkylene, $C_3$–$C_{12}$alkylene substituted by a hydroxyl group or interrupted by an oxygen atom, $C_4$–$C_{12}$alkenylene or $C_8$–$C_{12}$aralkylene, $R_4$ is $C_2$–$C_{18}$diacyl or a group of the formula VIII

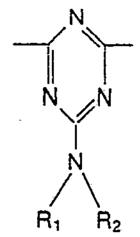

(VIII)

in which $R_1$ and $R_2$ are as defined above, a is a number between 0.2 and 1, and b is a number between zero and 0.8, where the compounds of the formula I have an average molecular weight $\overline{M}n$ between 1,000 and 20,000.

2. A compound according to claim 1, of the formula I, in which $R_1$ and $R_2$ independently of one another are hydrogen, $C_1$–$C_{12}$alkyl, 2-methoxyethyl, 2-ethoxyethyl, 3-methoxypropyl, 3-ethoxypropyl, 3-octyloxypropyl, 3-dodecyloxypropyl, $C_3$–$C_6$alkenyl, $C_6$–$C_{10}$cycloalkyl or a group of the formula II in which $R_6$ is hydrogen, methyl, allyl, benzyl or acetyl, or $R_1$ and $R_2$, together with the nitrogen atom to which they are bonded, form pyrrolidin-1-yl, piperidin-1-yl, hexahydroazepin-1-yl or morpholin-4-yl, $R_3$ and $R_5$ are a group of the formula III and $R_5$ additionally can be a group of the formula VII, $R_7$ is hydrogen, methyl, or ethyl, Y is $C_2$–$C_6$alkylene, but-2-ene-1,4-diyl or xylylene, $R_4$ is $C_2$–$C_{12}$diacyl or a group of the formula VIII in which $R_1$ and $R_2$ are as defined above, a is a number between 0.3 and 1, and b is a number between zero and 0.7, where the compounds of the formula I have an average molecular weight $\overline{M}n$ between 1,500 and 10,000.

3. A compound according to claim 1, of the formula I, in which $R_1$ and $R_2$ independently of one another are $C_1$–$C_8$alkyl, cyclohexyl, 2,2,6,6-tetramethyl-piperidin-4-yl or 1,2,2,6,6-pentamethylpiperidin-4-yl, $R_3$ and $R_5$ are a group of the formula III and $R_5$ additionally can be a group of the formula VII, $R_7$ is hydrogen or methyl, Y is but-2-ene-1,4-diyl or xylyene, a is the number 1 and b is zero, where the compounds of the formula I have an average molecular weight $\overline{M}n$ between 1,500 and 6,000.

4. A polymer composition which contains a synthetic polymer and stabiliser compound of the formula I, according to claim 1, in a quantity between 0.01 and 5% by weight, relative to the weight of the synthetic polymer.

5. A composition according to claim 4, which, in addition to the novel stabiliser contains other conventional additives for synthetic polymers.

6. A composition according to claim 4, wherein the synthetic polymer is polyethylene or polypropylene.

**Revendications**

1. Composés qui répondent à la formule I:

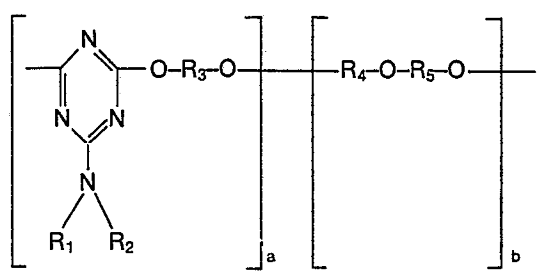

(I)

dans laquelle:

$R_1$ et $R_2$ représentent chacun, indépendamment l'un de l'autre, l'hydrogène, un alkyle en $C_1$–$C_{18}$, un radical hydroxy-2 éthyle, hydroxy-3 propyle, méthoxy-2 éthyle, éthoxy-2 éthyle, méthoxy-3 propyle, éthoxy-3 propyle, octyloxy-3 propyle, dodécyloxy-3 propyle, octadécyloxy-3 propyle, diméthylamino-3 propyle, diéthylamino-3 propyle ou diéthylamino-4 butyle, un alcényle en $C_3$–$C_{18}$, un cycloalkyle en $C_5$–$C_{18}$, un aryle en $C_6$–$C_{18}$, un aralkyle en $C_7$–$C_{18}$ ou un radical de formule II :

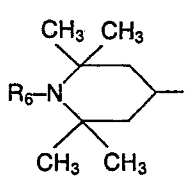

(II)

dans lequel $R_6$ représente l'hydrogène, un alkyle en $C_1$–$C_{12}$, un alcényle en $C_3$–$C_{12}$, un alcynyle en $C_3$–$C_{12}$, un aralkyle en $C_7$–$C_{12}$ ou un acyle en $C_1$–$C_{12}$, ou

$R_1$ et $R_2$ forment ensemble, et avec l'atome d'azote auxquels ils sont liés, un radical hétérocyclique pris dans l'ensemble constitué par les radicaux pyrrolidinyle-1, pipéridyle-1, hexahydroazépinyle-1, morpholinyle-4 et méthyl-4 pipérazinyle-1,

$R_3$ et $R_4$ représentent chacun, indépendamment l'un de l'autre, un radical répondant à l'une des formules III, IV, V et VI:

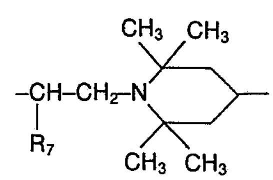

(III)

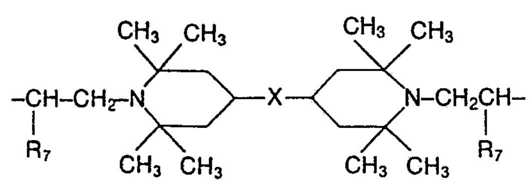

(IV)

Die folgenden Beispiele sollen der besseren Veranschaulichung de vorliegenden Erfindung dienen und bedeuten keinerlei Einschränkung der Erfindung.

Beispiel 1:
35,1 g (0,1 Mol)
2-[N-(2,2,6,6-Tetramethylpiperidin-4-yl)-butyl-amino]-4,6-dimethoxy-1,3,5-triazin,
20,1 g (0,1 Mol) 1-(2-Hydroxyethyl)-2,2,6,6-tetramethylpiperidin-4-ol, 0,3 g Natriumhydrid und 200 ml Xylol werden während 12 Stunden unter Rückfluss erhitzt. Das Lösungsmittel wird dabei langsam, zusammen mit dem bei der Umsetzung sich bildenden Methanol, abdestilliert und mit der gleichen Volumenmenge an frischem Lösungsmittel ersetzt. Das Reaktionsgemisch wird zur Trockne abgedampft und der Rückstand gemahlen, mit Wasser gewaschen und getrocknet.
Man erhält ein Produkt mit Smp. 205–215 °C und mittlerem Molekulargewicht $\overline{M}n$ 4300.

Beispiele 2 und 3:
Wiederholt man das unter Beispiel 1 beschriebene Verfahren mit den in der nachfolgenden Tabelle auch mengenmässig angegebenen Edukten, so erhält man entsprechende Verbindungen der Formel I

| Beispiel | Edukte | | Produkt $\overline{M}n$ | Smp. |
|---|---|---|---|---|
| 2 | 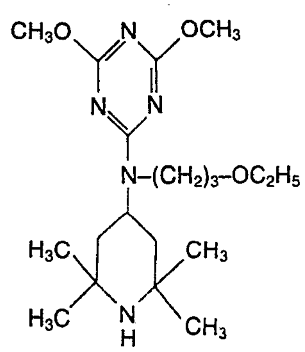 38,1 g (0,1 Mol) | 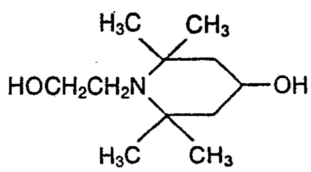 20,1 g (0.1 Mol) | 3200 | 160–172° C |
| 3 | 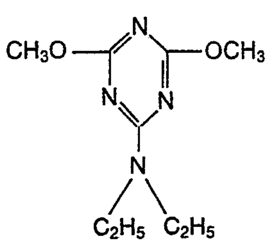 21,2 g (0.1 Mol) | 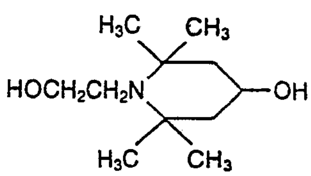 20,1 g (0,1 Mol) | 1800 | 152–160° C |

Beispiel 4:
26,8 g (0,1 Mol) 4-Octylamino-2,2,6,6-Tetramethylpiperidin werden langsam einer auf 0 °C abgekühlten Lösung von 18,5 g (0,1 Mol) Cyanurchlorid in 180 ml Trimethylbenzol zugegeben, wobei die Temperatur 10 °C nicht übersteigen darf. Danach wird bei der gleichen Temperatur eine Lösung von 4 g Natriumhydrid in 50 ml Wasser zugesetzt. Dem so erhaltenen Gemisch werden 20,1 g (0,1 Mol) 1-(2-Hydroxyethyl)-2,2,6,6-tetramethylpiperidin-4-ol und 12 g Natriumhydroxid beigefügt. Das Gemisch wird 20 Stunden unter Rückfluss gekocht und das Wasser azeotrop entfernt.

Nach dem Filtrieren, zwecks Abtrennung der anorganischen Produkte, und nach dem Abdestillieren des Lösungsmittels erhält man ein Produkt mit Smp. 145–152 °C und mittlerem Molekulargewicht $\overline{M}n$ 2200.

Beispiele 5 und 6:
Wiederholt man das unter Beispiel 4 beschriebene Verfahren mit den in der nachfolgenden Tabelle auch mengenmässig angegebenen Edukten, so erhält man entsprechende Verbindungen der Formel I.

| Beispiel | Edukte | | | Produkt $\overline{Mn}$ | Smp. |
|---|---|---|---|---|---|
| 5 | 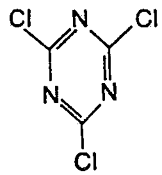 | 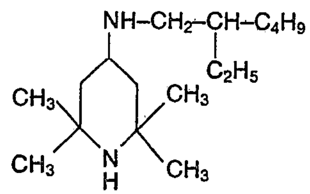 | 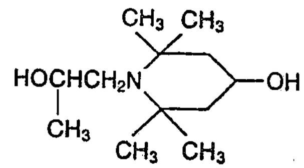 | 1600 | 125–136° C |
| | 18,5 g (0,1 Mol) | 26,8 g (0,1 Mol) | 21,5 g (0,1 Mol) | | |
| 6 | 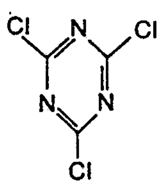 | 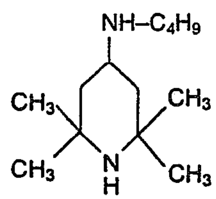 | 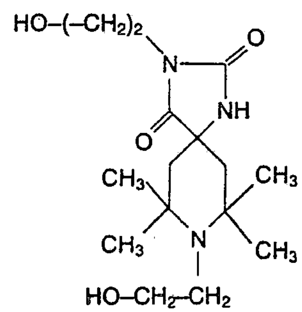 | 2450 | 210–220° C |
| | 18,5 g (0,1 Mol) | 21,2 g (0,1 Mol) | 31,2 g (0,1 Mol) | | |

Beispiel 7:

20,4 g (0,05 Mol)
2,4-Dimethoxy-6-[N-(2,2,6,6-Tetramethyl-piperidin-4-yl)-octylamino]-1,3,5-triazin,
20,1 g (0,1 Mol)
1-(2-Hydroxyethyl)-2,2,6,6-Tetramethylpiperidin-4-ol,
0,25 g Natrium und 200 ml Xylol werden 8 Stunden unter Rückfluss gekocht. Das Lösungsmittel wird dabei langsam, zusammen mit dem bei der Umsetzung sich bildenden Methanol, abdestilliert und mit der gleichen Volumenmenge an frischem Lösungsmittel ersetzt.

Das Gemisch wird auf 80 °C abgekühlt, dann werden 7,3 g (0,05 Mol) Dimethylsuccinat zugefügt und weitere 8 Stunden am Rückfluss gekocht, bei fortdauerndem Ersatz des Lösungsmittels.

Nach Reaktionsende wird das Gemisch mit 200 ml Xylol verdünnt, filtriert und das Filtrat zur Trockne eingedampft. Das erhaltene Produkt schmilzt bei 95–103 °C und hat ein mittleres Molekulargewicht $\overline{M}n$ von 3700.

Beispiel 8:

Wiederholt man das in Beispiel 7 beschriebene Verfahren mit 17,55 g (0,05 Mol)
2-[N-(2,2,6,6-Tetramethyl-piperidin-4-yl)-butylamino]-4,6-dimethoxy-1,3,5-triazin,
20,1 g (0,1 Mol)
1-(2-Hydroxyethyl)-2,2,6,6-Tetramethyl-piperidin-4-ol
und 7,3 g (0,05 Mol) Dimethylsuccinat als Ausgangsprodukte, so erhält man ein Produkt mit Smp. 125–134 °C und mittlerem Molekulargewicht $\overline{M}n$ 2000.

Beispiel 9:

Je 2 g der in Tabelle 1 angeführten Verbindungen und 1 g
Pentaerythrit-tetrakis-3-(3,5-di-t-butyl-4-hydroxyphenyl)-propionat (Antioxidans)
werden mit 1000 g Polypropylen mit einem Schmelzindex von 2,4 (®Propathen HF 18, Produkt der Imperial Chemical Industries) und 1 g Calciumstearat innig vermischt.

Das erhaltene Gemisch wird dann bei einer Temperatur von 180 bis 220 °C extrudiert und zu Granulat verarbeitet, aus dem man Bänder einer Dicke von 50 µm und einer Breite von 2,5 mm herstellt. Die Arbeitsbedingungen sind:

| | |
|---|---|
| Extrudertemperatur: | 220 bis 240 °C |
| Kopftemperatur: | 240 °C |
| Streckverhältnis: | 1 : 6 |

die erhaltenen Bänder werden auf weisser Pappe angeordnet in einem Weather-Ometer 65 WR(ASTM G 27–70) bei einer Temperatur der schwarzen Tafel von 63 °C ausgesetzt. Von Zeit zu Zeit werden Proben entnommen, an denen man die verbleibende Zugfestigkeit mittels eines Tensometers mit konstanter Geschwindigkeit misst; danach bestimmt man die zum Halbieren der ursprünglichen Zugfestigkeit erforderliche Aussetzungszeit ($T_{50}$).

Die Resultate sind in Tabelle 1 wiedergegeben.

Tabelle 1

| Stabilisator | $T_{50}$ (Stunden) |
|---|---|
| Keiner | 230 |
| Verbindung von Beispiel 4 | 2 370 |
| Verbindung von Beispiel 5 | 1 600 |
| Verbindung von Beispiel 7 | 1 800 |

Beispiel 10:

Je 2,5 g der in Tabelle 2 angeführten Verbindungen und 1 g
n-Octadecyl-3-(3,5-di-t-butyl-4-hydroxyphenyl)-propionat
(Antioxidans) werden mit 1000 g Polypropylen mit einem Schmelzindex von 13 (®Propathen HF 85, Produkt der Imperial Chemical Industries), 1 g Calciumstearat und 2,5 g Titandioxid (®KRONOS RN 57) innig vermischt.

Das erhaltene Gemisch wird dann bei einer Temperatur von 180 bis 220 °C extrudiert und zu Granulat verarbeitet. Die so erhaltenen Granulate werden unter folgenden Bedingungen zu Fasern (Titer: 22dtex/Faser) verarbeitet:

| | |
|---|---|
| Extrudertemperatur: | 220 bis 240 °C |
| Kopftemperatur: | 240 °C |
| Streckverhältnis: | 1 : 3,5 |

Die erhaltenen Fasern werden auf weisser Pappe angeordnet in einem Weather-Ometer 65 WR bei einer Temperatur der schwarzen Tafel von 63 °C ausgesetzt. Der $T_{50}$-Wert wird wie unter Beispiel 9 beschrieben bestimmt.

Die Resultate sind in Tabelle 2 wiedergegeben.

Tabelle 2

| Stabilisator | $T_{50}$ (Stunden) |
|---|---|
| keiner | 120 |
| Verbindung von Beispiel 4 | 1 070 |
| Verbindung von Beispiel 5 | 1 110 |
| Verbindung von Beispiel 7 | 1 050 |
| Verbindung von Beispiel 8 | 1 370 |

**Patentansprüche**

1. Verbindungen der Formel I

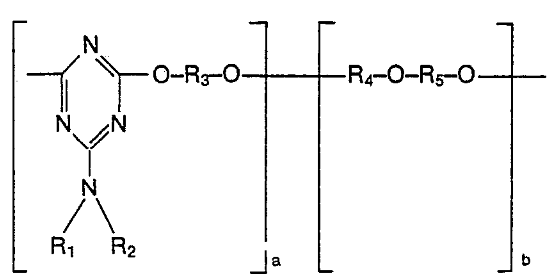

(I)

worin $R_1$ und $R_2$ unabhängig voneinander Wasserstoff, $C_1$–$C_{18}$ Alkyl, 2-Hydroxyethyl, 3-Hydroxypropyl, 2-Methoxyethyl, 2-Ethoxyethyl, 3-Methoxypropyl, 3-Ethoxypropyl, 3-Octyloxypropyl, 3-Dodecyloxypropyl,

3-Octadecyloxypropyl, 3-Dimethylaminopropyl, 3-Diethylaminopropyl, 4-Diethylaminobutyl, $C_3$–$C_{18}$ Alkenyl, $C_5$–$C_{18}$ Cycloalkyl, $C_6$–$C_{18}$ Aryl, $C_7$–$C_{18}$ Aralkyl oder eine Gruppe der Formel II

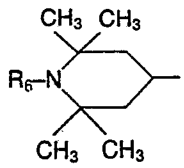
(II)

bedeuten, worin $R_6$ Wasserstoff, $C_1$–$C_{12}$ Alkyl, $C_3$–$C_{12}$ Alkenyl oder Alkynyl, $C_7$–$C_{12}$ Aralkyl oder $C_1$–$C_{12}$ Acyl ist, oder $R_1$ und $R_2$ zusammen mit dem Stickstoffatom, an dem sie gebunden sind, einen heterocyclischen Rest ausgewählt aus der Gruppe bestehend aus Pyrrolidin-1-yl, Piperidin-1-yl, Hexahydroazepin-1-yl, Morpholin-4-yl und 4-Methylpiperazin-1-yl bilden, $R_3$ und $R_5$ unabhängig voneinander eine der Gruppen der Formeln III, IV, V oder VI

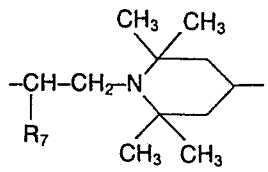
(III)

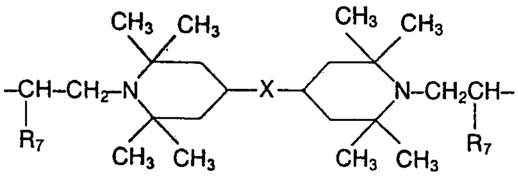
(IV)

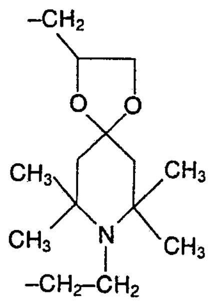
(V)

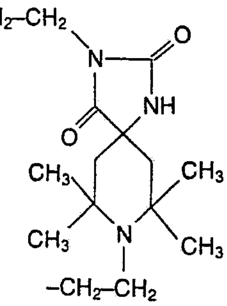
(VI)

bedeuten und $R_5$ zusätzlich eine gruppe der Formel VII

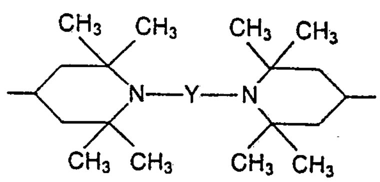
(VII)

sein kann, worin $R_7$ Wasserstoff, $C_1$–$C_6$ Alkyl oder Phenyl ist, X eine Gruppe $-O-(CH_2)_n-O-$ oder $-N(R_8)-C(O)-(CH_2)_n-C(O)-N(R_8)-$ bedeutet, n eine Zahl von 1 bis 6 ist, $R_8$ die gleiche Bedeutung wie $R_1$ und $R_2$ hat und Y $C_2$–$C_{12}$ Alkylen, durch eine Hydroxygruppe substituiertes oder durch ein Sauerstoffatom unterbrochenes $C_3$–$C_{12}$ Alkylen, $C_4$–$C_{12}$ Alkenylen oder $C_8$–$C_{12}$ Aralkylen bedeutet, $R_4$ $C_2$–$C_{18}$ Diacyl oder eine Gruppe der Formel VIII

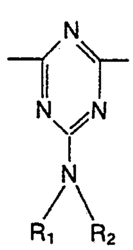
(VIII)

ist, worin $R_1$ und $R_2$ die oben angegebene Bedeutung haben, a eine Zahl zwischen 0,2 und 1 und b eine Zahl zwischen Null und 0,8 sind, wobei die Verbindungen der Formel I ein mittleres Molekulargewicht $\overline{M}n$ zwischen 1000 und 20 000 aufweisen.

2. Verbindungen gemäss Anspruch 1, der Formel I, worin $R_1$ und $R_2$ unabhängig voneinander Wasserstoff, $C_1$–$C_{12}$ Alkyl, 2-Methoxyethyl, 2-Ethoxyethyl, 3-Methoxypropyl, 3-Ethoxypropyl, 3-Octyloxypropyl, 3-Dodecyloxypropyl, $C_3$–$C_6$ Alkenyl, $C_6$–$C_{10}$ Cycloalkyl oder eine Gruppe der Formel II bedeuten, worin $R_6$ Wasserstoff, Methyl, Allyl, Benzyl oder Acetyl ist, oder $R_1$ und $R_2$ zusammen mit dem Stickstoffatom an dem sie gebunden sind Pyrrolidin-1-yl, Piperidin-1-yl, Hexahydroazepin-1-yl oder Morpholin-4-yl bilden, $R_3$ und $R_5$ eine Gruppe der Formel III sind und $R_5$ zusätzlich eine Gruppe der Formel VII sein kann, $R_7$ Wasserstoff, Methyl oder Ethyl ist, Y $C_2$–$C_6$ Alkylen, But-2-en-1,4-diyl oder Xylylen bedeutet, $R_4$ $C_2$–$C_{12}$ Diacyl oder eine Gruppe der Formel VIII ist, worin $R_1$ und $R_2$ die oben angegebene Bedeutung haben, a eine Zahl zwischen 0,3 und 1 und b eine Zahl zwischen Null und 0,7 bedeuten, wobei die Verbindungen der Formel I ein mittleres Molekulargewicht $\overline{M}n$ zwischen 1500 und 10 000 aufweisen.

3. Verbindungen gemäss Anspruch 1, der Formel I, worin $R_1$ und $R_2$ unabhängig voneinander $C_1$–$C_8$ Alkyl, Cyclohexyl, 2,2,6,6-Tetramethylpiperidin-4-yl oder 1,2,2,6,6-Pentamethylpiperidin-4-yl bedeuten, $R_3$ und $R_5$ eine Gruppe der Formel III sind und $R_5$ zusätzlich eine Gruppe der Formel VII

beispielsweise Antioxydantien, UV-absorbierende Stoffe, Nickelstabilisatoren, Pigmente, Füllstoffe, Weichmacher, Antistatika, Flammschutzmittel, Schmierstoffe, Korrosionshemmstoffe sowie Metalldesaktivatoren. Beispiele für in Mischung mit Verbindungen der Formel I verwendbare Zusatzstoffe sind insbesondere:

1. Antioxidantien
1.1. Alkylierte Monophenole
2,6-Di-tert.butyl-4-methylphenol
2-Tert.butyl-4,6-dimethylphenol
2,6-Di-tert.butyl-4-ethylphenol
2,6-Di-tert.butyl-4-n-butylphenol
2,6-Di-tert.butyl-4-i-butylphenol
2,6-Di-cyclopentyl-4-methylphenol
2-(α-Methylcyclohexyl)-4,6-dimethylphenol
2,6-Di-octadecyl-4-methylphenol
2,4,6-Tri-cyclohexylphenol
2,6-Di-tert.butyl-4-methoxymethylphenol

1.2. Alkylierte Hydrochinone
2,6-Di-tert.butyl-4-methoxyphenol
2,5-Di-tert.butyl-hydrochinon
2,5-Di-tert.amyl-hydrochinon
2,6-Diphenyl-4-octadecyloxyphenol

1.3. Hydroxylierte Thiodiphenylether
2,2′-Thio-bis-(6-tert.butyl-4-methylphenol)
2,2′-Thio-bis-(4-octylphenol)
4,4′-Thio-bis-(6-tert.butyl-3-methylphenol)
4,4′-Thio-bis-(6-tert.butyl-2-methylphenol)

1.4. Alkyliden-Bisphenole
2,2′-Methylen-bis-(6-tert.butyl-4-methylphenol)
2,2′-Methylen-bis-(6-tert.butyl-4-ethylphenol)
2,2′-Methylen-bis-[4-methyl-6-(α-methylcyclohexyl)-phenol]
2,2′-Methylen-bis-(4-methyl-6-cyclohexylphenol)
2,2′-Methylen-bis-(6-nonyl-4-methylphenol)
2,2′-Methylen-bis-(4,6-di-tert.butylphenol)
2,2′-Ethyliden-bis-(4,6-di-tert.butylphenol)
2,2′-Ethyliden-bis-(6-tert.butyl-4-isobutylphenol)
2,2′-Methylen-bis-[6-(α-methylbenzyl)-4-nonylphenol]
2,2′-Methylen-bis-[6-(α,α-dimethylbenzyl)-4-nonylphenol]
4,4′-Methylen-bis-(2,6-di-tert.butylphenol)
4,4′-Methylen-bis-(6-tert.butyl-2-methylphenol)
1,1-Bis-(5-tert.butyl-4-hydroxy-2-methylphenyl)-butan
2,6-Di-(3-tert.butyl-5-methyl-2-hydroxybenzyl)-4-methylphenol
1,1,3-Tris-(5-tert.butyl-4-hydroxy-2-methylphenyl)-butan
1,1-Bis-(5-tert.butyl-4-hydroxy-2-methylphenyl)-3-n-dodecylmercaptobutan
Ethylenglycol-bis-[3,3-bis-(3′-tert.butyl-4′-hydroxyphenyl)-butyrat]
Di-(3-tert.butyl-4-hydroxy-5-methylphenyl)-dicyclopentadien
Di-[2-(3′-tert.butyl-2′-hydroxy-5′-methyl-benzyl)-6-tert.butyl-4-methyl-phenyl]-terephthalat.

1.5. Benzylverbindungen
1,3,5-Tri-(3,5-di-tert.butyl-4-hydroxybenzyl)-2,4,6-trimethylbenzol
Di-(3,5-di-tert.butyl-4-hydroxybenzyl)-sulfid
3,5-di-tert.butyl-4-hydroxybenzyl-mercaptoessigsäure-isooctylester
Bis-(4-tert.butyl-3-hydroxy-2,6-dimethylbenzyl)-dithiol-terephthalat
1,3,5-Tris-(3,5-di-tert.butyl-4-hydroxybenzyl)-isocyanurat
1,3,5-Tris-(4-tert.butyl-3-hydroxy-2,6-dimethylbenzyl)-isocyanurat
3,5-Di-tert.butyl-4-hydroxybenzyl-phosphonsäure-dioctadecylester
3,5-Di-tert.butyl-4-hydroxybenzyl-phosphonsäure-monoethylester,
Calciumsalz.

1.6. Acylaminophenole
4-Hydroxy-laurinsäureanilid
4-Hydroxy-stearinsäureanilid
2,4-Bis-octylmercapto-6-(3,5-di-tert.butyl-4-hydroxyanilino)-s-triazin
N-(3,5-di-tert.butyl-4-hydroxyphenyl)-carbaminsäureoctylester.

1.7. Ester der β-(3,5-Di-tert.butyl-4-hydroxyphenyl)-propionsäure
mit ein- oder mehrwertigen Alkoholen, wie z.B. mit
Methanol, Octadecanol, 1,6-Hexandiol,
Neopentylglycol, Thiodiethylenglycol, Diethylenglycol,
Triethylenglycol, Pentaerythrit,
Tris-hydroxyethyl-isocyanurat,
Di-hydroxyethyl-oxalsäurediamid

1.8. Ester der β-(5-tert.butyl-4-hydroxy-3-methylphenyl)-propionsäure
mit ein- oder mehrwertigen Alkoholen, wie z.B. mit
Methanol, Octadecanol, 1,6-Hexandiol,
Neopentylglycol, Thiodiethylenglycol,
Diethylenglycol, Triethylenglycol, Pentaerythrit,
Tris-hydroxyethyl-isocyanurat,
Di-hydroxyethyl-oxalsäurediamid

1.9. Amide der β-(3,5-Di-tert.butyl-4-hydroxyphenyl)-propionsäure,
wie z.B.
N,N′-Di-(3,5-di-tert.butyl-4-hydroxyphenylpropionyl)-hexamethylendiamin
N,N′-Di-(3,5-di-tert.butyl-4-hydroxyphenylpropionyl)-trimethylendiamin
N,N′-Di-(3,5-di-tert.butyl-4-hydroxyphenylpropionyl)-hydrazin

2. UV-Absorber und Lichtschutzmittel
2.1. 2-(2′-Hydroxyphenyl)-benztriazole,
wie z.B. das
5′-Methyl-, 3′,5′-Di-tert.butyl-, 5′-Tert.butyl-,
5′-(1,1,3,3-Tetramethylbutyl)-,
5-Chlor-3′,5′-di-tert.butyl-,
5-Chlor-3′-tert.butyl-5′-methyl-,
3′-sec.Butyl-5′-tert.butyl, 4′-Octoxy-,

3′,5′-Di-tert.amyl-,
3′,5′-Bis-(α,α-dimethylbenzyl)-Derivat.

2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2′,4′-Trihydroxy-, 2′-Hydroxy-4,4′-dimethoxy-Derivat.

2.3. Ester von gegebenenfalls substituierten Benzoesäuren, wie z.B.
4-Tert.butyl-phenylsalicylat, Phenylsalicylat, Octylphenylsalicylat, Dibenzoylresorcin, Bis-(4-tert.butylbenzoyl)-resorcin, Benzoylresorcin,
3,5-Di-tert.butyl-4-hydroxybenzoesäure-2,4-di-tert.butylphenylester,
3,5-Di-tert.butyl-4-hydroxybenzoesäurehexadecylester.

2.4. Acrylate, wie z.B.
α-Cyan-β,β-diphenylacrylsäure-ethylester bzw. isooctylester,
α-Carbomethoxy-zimtsäuremethylester,
α-Cyano-β-methyl-p-methoxy-zimtsäuremethylester bzw. -butylester,
α-Carbomethoxy-p-methoxy-zimtsäure-methylester,
N-(β-Carbomethoxy-β-cyanovinyl)-2-methylindolin.

2.5. Nickelverbindungen, wie z.B. Nickelkomplexe des 2,2′-Thio-bis-[4-(1,1,3,3-tetramethylbutyl)-phenols], wie der 1:1- oder der 1:2-Komplex, gegebenenfalls mit zusätzlichen Liganden wie n-Butylamin, Triethanolamin oder N-Cyclohexyl-diethanolamin, Nickeldibutyldithiocarbamat, Nikkelsalze von 4-Hydroxy-3,5-di-tert.butylbenzyl-phosphonsäure-monoalkylestern wie vom Methyl- oder Ethylester, Nickelkomplexe von Ketoximen wie von 2-Hydroxy-4-methyl-phenyl-undecylketonoxim, Nickelkomplexe des 1-Phenyl-4-lauroyl-5-hydroxy-pyrazols, gegebenenfalls mit zusätzlichen Liganden.

2.6. Sterisch gehinderte Amine, wie z.B.
Bis-(2,2,6,6-tetramethylpiperidyl)-sebacat
Bis-(1,2,2,6,6-pentamethylpiperidyl)-sebacat
n-Butyl-3,5-di-tert.butyl-4-hydroxybenzyl-malonsäure-bis(1,2,2,6,6-pentamethylpiperidyl)-ester,
Kondensationsprodukt aus
1-Hydroxyethyl-2,2,6,6-tetramethyl-4-hydroxy-piperidin und Bernsteinsäure,
Kondensationsprodukt aus
N,N′-(2,2,6,6-Tetramethyl-4-piperidyl)-hexamethylendiamin und
4-tert.Octylamino-2,6-dichlor-1,3,5-s-triazin,
Tris-(2,2,6,6-tetramethyl-4-piperidyl)-nitrilotriacetat,
Tetrakis-(2,2,6,6-tetramethyl-4-piperidyl)-1,2,3,4-butantetracarbonsäure,
1,1′-(1,2-Ethandiyl)-bis-(3,3,5,5-tetramethyl-piperazinon).

2.7. Oxalsäurediamide, wie z.B.
4,4′-Di-octyloxy-oxanilid,
2,2′-Di-octyloxy-5,5′-di-tert.butyl-oxanilid,
2,2′-Di-dodecyloxy-5,5′-di-tert.butyl-oxanilid,
2-Ethoxy-2′-ethyl-oxanilid,
N,N′-Bis-(3-dimethylaminopropyl)-oxalamid,
2-Ethoxy-5-tert.butyl-2′-ethyl-oxanilid
und dessen Gemisch mit 2-Ethoxy-2′-ethyl-5,4′-di-tert.butyl-oxanilid, Gemische von ortho- und para-Methoxy- sowie von o- und p-Ethoxy-di-substituierte Oxaniliden.

3. Metalldesaktivatoren, wie z.B.
N,N′-Diphenyloxalsäurediamid,
N-Salicylal-N′-salicyloylhydrazin,
N,N′-Bis-salicyloylhydrazin,
N,N′-Bis-(3,5-di-tert.butyl-4-hydroxyphenylpropionyl)-hydrazin,
3-Salicyloylamino-1,2,4-triazol,
Bis-benzyliden-oxalsäuredihydrazid.

4. Phosphite und Phosphonite, wie z.B.
Triphenylphosphit, Diphenylalkylphosphite, Phenyldialkylphosphite,
Tri-(nonylphenyl)-phosphit, Trilaurylphosphit,
Trioctadecylphosphit,
Distearyl-pentaerythritdiphosphit,
Tris-(2,4-di-tert.butylphenyl)-phosphit,
Diisodecylpentaerythrit-diphosphit,
Di-(2,4-di-tert.butylphenyl)-pentaerythritdiphosphit,
Tristearyl-sorbit-triphosphit,
Tetrakis-(2,4-di-tert.butylphenyl)-4,4′-biphenylen-diphosphonit.

5. Peroxidzerstörende Verbindungen, wie z.B Ester der β-Thio-dipropionsäure, beispielsweise der Lauryl-, Stearyl-, Myristyl- oder Tridecylester Mercaptobenzimidazol, das Zinksalz des 2-Mer captobenzimidazols, Zink-dibutyl-dithiocarbamat Dioctadecyldisulfid, Pentaerythrit-tetrakis-(β dodecylmercapto)-propionat.

6. Polyamidstabilisatoren, wie z.B. Kupfersalze i Kombination mit Jodiden und/oder Phosphorver bindungen und Salze des zweiwertigen Mangans

7. Basische Co-Stabilisatoren, wie z.B. Melamir Polyvinylpyrrolidon, Dicyandiamid, Triallylcyanu rat, Harnstoff-Derivate, Hydrazin-Derivate, Amine Polyamide, Polyurethane, Alkali- und Erdalkal salze höherer Fettsäuren, beispielsweise Ca Stearat, Zn-Stearat, Mg-Stearat, Na-Ricinoleat, K Palmitat, Antimonbrenzcatechinat oder Zinn brenzcatechinat.

8. Nukleierungsmittel, wie z.B. 4-tert.Butylbenzoe säure, Adipinsäure, Diphenylessigsäure.

9. Füllstoffe und Verstärkungsmittel, wie z.B. Ca ciumcarbonat, Silikate, Glasfasern, Asbest, Tal Kaolin, Glimmer, Bariumsulfat, Metalloxide ur -hydroxide, Russ, Graphit.

10. Sonstige Zusätze, wie z.B. Weichmache Gleitmittel, Emulgatoren, Pigmente, Optische Au heller, Flammschutzmittel, Antistatika, Treibm tel.

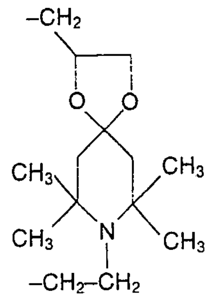

(V)

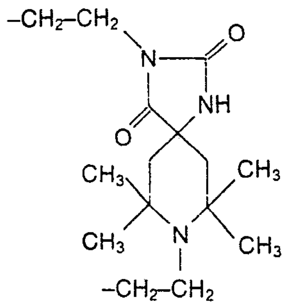

(VI)

et $R_5$ peut en outre représenter un radical de formule VII:

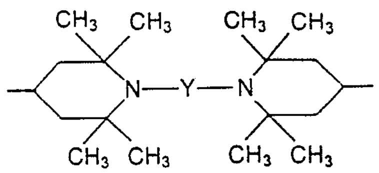

(VII)

radicaux dans lesquels $R_7$ représente l'hydrogène, un alkyle en $C_1$–$C_6$ ou un phényle, X représente un radical $-O-(CH_2)_n$ O– ou $-N(R_8)-C(O)-CH_2)_n$ $C(O)-N(R_8)-$, n désigne un nombre de 1 à 6, $R_8$ a la même signification que $R_1$ et $R_2$, et Y représente un alkylène en $C_2$–$C_{12}$, un alkylène en $C_3$–$C_{12}$ interrompu par un atome d'oxygène ou porteur d'un radical hydroxy, un alcénylène en $C_4$–$C_{12}$ ou un aralkylène en $C_8$–$C_{12}$,

$R_4$ représente un diacyle en $C_2$–$C_{18}$ ou un radical de formule VIII:

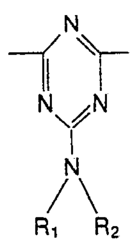

(VIII)

dans lequel $R_1$ et $R_2$ ont les significations qui leur ont été données ci-dessus,

a désigne un nombre compris entre 0,2 et 1 et

b désigne un nombre compris entre 0 et 0,8,

et dont la masse moléculaire moyenne $\overline{M}n$ est comprise entre 1000 et 20 000.

2. Composés de formule I selon la revendication 1, dans lesquels:

$R_1$ et $R_2$ représentent chacun, indépendamment l'un de l'autre, l'hydrogène, un alkyle en $C_1$–$C_{12}$, un radical méthoxy-2 éthyle, éthoxy-2 éthyle, méthoxy-3 propyle, éthoxy-3 propyle, octyloxy-3 propyle ou dodécyloxy-3 propyle, un alcényle en $C_3$–$C_6$, un cycloalkyle en $C_6$–$C_{10}$ ou un radical de formule II dans lequel $R_6$ représente l'hydrogène ou un radical méthyle, allyle, benzyle ou acétyle, ou $R_1$ et $R_2$ forment ensemble, et avec l'atome d'azote auquel ils sont liés, un radical pyrrolidinyle-1, pipéridyle-1, hexahydroazépinyle-1 ou morpholinyle-4, $R_3$ et $R_5$ représentent chacun un radical de formule III, $R_5$ peut en outre représenter un radical de formule VII, $R_7$ représente l'hydrogène ou un radical méthyle ou éthyle, Y représente un alkylène en $C_2$–$C_6$, un butène-2 diyle-1,4 ou un xylylène, $R_4$ représente un diacyle en $C_2$–$C_{12}$ ou un radical de formule VIII dans lequel $R_1$ et $R_2$ ont les significations précédemment données, a représente un nombre compris entre 0,3 et 1, et b représente un nombre compris entre 0 et 0,7,

et dont la masse moléculaire moyenne $\overline{M}n$ est comprise entre 1500 et 10 000.

3. Composés de formule I selon la revendication 1 dans lesquels:

$R_1$ et $R_2$ représentent chacun, indépendamment l'un de l'autre, un alkyle en $C_1$–$C_8$ ou un radical cyclohexyle, tétraméthyl-2,2,6,6 pipéridyle-4 ou pentaméthyl-1,2,2,6,6 pipéridyle-4, $R_3$ et $R_5$ représentent chacun un radical de formule III, $R_5$ peut en outre représenter un radical de formule VII, $R_7$ représente l'hydrogène ou un méthyle, Y représente un radical butène-2 diyle-1,4 ou xylylène, a est égal à 1 et b est égal à 0,

et dont la masse moléculaire moyenne $\overline{M}n$ est comprise entre 1500 et 6000.

4. Compositions polymères caractérisées en ce qu'elles contiennent un polymère synthétique et un composé stabilisant de formule I selon la revendication 1 en une quantité comprise entre 0,01 et 5% en poids par rapport au poids du polymère synthétique.

5. Compositions selon la revendication 4 caractérisées en ce qu'elles contiennent, en plus du nouveau stabilisant, des stabilisants usuels pour polymères synthétiques.

6. Compositions selon la revendication 4 caractérisées en ce qu'elles contiennent, comme polymère synthétique, du polyéthylène ou du polypropylène.